(19) Europäisches Patentamt
European Patent Office
Office européen des brevets

(11) Publication number: **0 539 588 A1**

# (12) EUROPEAN PATENT APPLICATION published in accordance with Art. 158(3) EPC

(21) Application number: **91912066.7**

(22) Date of filing: **02.07.91**

(86) International application number: **PCT/JP91/00889**

(87) International publication number: **WO 92/00964 (23.01.92 92/03)**

(51) Int. Cl.[5]: **C07D 213/61**, C07D 213/64, C07D 237/08, C07D 237/12, C07D 277/28, C07D 277/32, C07D 401/12, C07D 403/12, C07D 405/12, C07D 409/12, C07D 417/12

(30) Priority: **05.07.90 JP 176462/90**
**09.08.90 JP 209238/90**
**28.02.91 JP 55562/91**

(43) Date of publication of application: **05.05.93 Bulletin 93/18**

(84) Designated Contracting States: **AT BE CH DE DK ES FR GB GR IT LI LU NL SE**

(71) Applicant: **NIPPON SODA CO., LTD.**
**2-1, Ohtemachi 2-chome**
**Chiyoda-ku, Tokyo 100(JP)**

(72) Inventor: **OHISHI, Haruhito, Odawara Research Center, Nippon Soda Co., Ltd. 345, Aza Yanagimachi Takada**
**Odawara-shi Kanagawa 250-02(JP)**
Inventor: **IIHAMA, Teruyuki, Odawara Research Center, Nippon Soda Co., Ltd. 345, Aza Yanagimachi Takada**
**Odawara-shi Kanagawa 250-02(JP)**
Inventor: **ISHIMITSU, Keiichi, Odawara Research Center,**
**Nippon Soda Co., Ltd. 345, Aza Yanagimachi Takada**
**Odawara-shi Kanagawa 250-02(JP)**
Inventor: **YAMADA, Tomio, Odawara Research Center,**
**Nippon Soda Co., Ltd. 345, Aza Yanagimachi Takada**
**Odawara-shi Kanagawa 250-02(JP)**
Inventor: **HATANO, Renpei, Odawara Research Center,**
**Nippon Soda Co., Ltd. 345, Aza Yanagimachi Takada**
**Odawara-shi Kanagawa 250-02(JP)**
Inventor: **TAKAKUSA, Nobuo, Odawara Research Center,**
**Nippon Soda Co., Ltd. 345, Aza Yanagimachi Takada**
**Odawara-shi Kanagawa 250-02(JP)**
Inventor: **MITSUI, Jun, Odawara Research Center,**
**Nippon Soda Co., Ltd. 345, Aza Yanagimachi Takada**
**Odawara-shi Kanagawa 250-02(JP)**

(74) Representative: **de Bruijn, Leendert C. (NL)**
**Nederlandsch Octrooibureau**
**Scheveningseweg 82 P.O. Box 29720**
**NL-2505 KZ Den Haag (NL)**

## (54) AMINE DERIVATIVE.

(57) A compound represented by general formula (I), its salt, production thereof, and insecticide containing the same wherein $R_1$ represents a 5- or 6-membered nitrogenous aromatic heterocyclic group which may be substituted; X represents alkylene or alkylidene; $R_2$ represents hydrogen, optionally substituted alkyl, alkenyl, alkynyl, cycloalkyl, cycloalkenyl, aryl or $-Y-R_3$; Y represents O, $S(O)_1$, CO or $CO_2$; 1 represents 0, 1 or 2; $R_3$ represents hydrogen, optionally substituted alkyl, alkenyl, alkynyl, cycloalkyl, cycloalkenyl or aryl; A, B and D

EP 0 539 588 A1

represent each independently an optionally substituted carbon atom or hetero-atom or a single bond; E represents CO or CS; Q represents hydrogen, optionally substituted alkyl, alkenyl, alkynyl, aryl, nitro, halogen or Z-$R_4$; Z represents CO, $CO_2$, or $S(O)_m$; m represents 0, 1 or 2; and $R_4$ represents optionally substituted alkyl, alkenyl, alkynyl, cycloalkyl, cycloalkenyl or aryl.

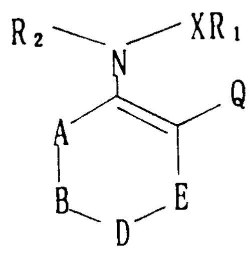

[ I ]

Field of the Invention

This invention relates to a novel amine derivative, preparation process thereof and an insecticide containing said amine derivative as an active ingredient.

Background of the Invention

By virtue of the research and development of insecticides for many years, a number of insecticides, for example organophosphorus insecticides such as parathion and malathion, and carbamate insecticides such as carbaryl, methomyl and the like, have been developed and practically used. Although the contribution of these insecticides for raising agricultural production yields should be considered as great, however among these insecticides, several cases such as the restriction of their use resulted from the problems of residues and accumulating pollution in the environment and occurrence of insecticide-resistant pests as a results of the use over long period.

Consequently, the development of novel insecticidal compounds which have excellent insecticidal property against insecticide-resistant pest insect species as well as other various pest insects and can be used safe is highly demanded.

The present invention has an object to provide an agricultural chemical which can be manufactured advantageously on the industrial basis and used safe as well as having firm insecticidal effectiveness.

Disclosure of the Invention

The present invention relates to a compound represented by the general formula [I] or its salts, the process for preparation thereof and an insecticide;

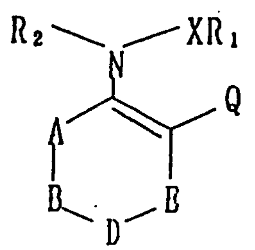

[I]

wherein $R_1$ represents unsubstituted or substituted aromatic 5- or 6-membered heterocyclic radical containing nitrogen: X represents unsubstituted or substituted alkylene or alkylidene;$R_2$ represents hydrogen, unsubstituted or substituted alkyl, alkenyl, cycloalkyl, cycloalkenyl, aryl or $-Y-R_3$; Y represents 0, $S(O)_1$, CO, $CO_2$; 1 represents 0, 1 or 2; and $R_3$ represents hydrogen, unsubstituted or substituted alkyl, alkenyl, alkynyl, cycloalkyl, cycloalkenyl or aryl. A, B and D represent each independently unsubstituted or substituted carbon atom, hetero atom or single bond; E represents CO or CS; Q represents hydrogen, unsubstituted or substituted alkyl, alkenyl, alkynyl, aryl, nitro, halogen atom or $Z-R_4$; Z represents CO, $CO_2$ or S(O)m; m is 0, 1 or 2: and $R_4$ represents unsubstituted or substituted alkyl, alkenyl, alkynyl, cycloalkyl, cycloalkenyl or aryl.

The compound specified in this invention is prepared by one of the processes (1)-(4) shown below.

(1)

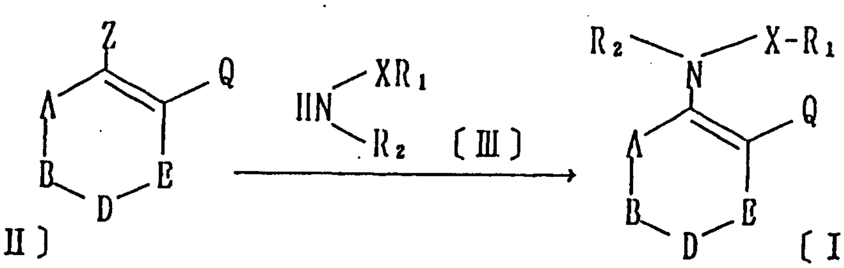

[II] [III] [I]

wherein $R_1$, $R_2$, X, A, B, D, E and Q represent the same meaning as described above. In the formula [II], Z represents $S(O)nR_5$, $OR_6$ or enolic hydroxy group; n is 0, 1 or 2; $R_5$ represents unsubstituted or substituted alkyl having 1 to 4 carbon atoms or phenyl; $R_6$ represents unsubstituted or substituted alkyl having 1 to 4 carbon atoms, phenyl or $SO_2R_7$; and $R_7$ represents unsubstituted or substituted alkyl having 1 to 4 carbon atoms or phenyl group.

The compound represented by the formula [I] can be prepared by reacting the compound represented by the formula [II] with amine represented by the formula [III] without solvent or in an inactive solvent such as benzene, toluene, ethanol, DMF, DME, N－methylpyrolidone or acetic acid, and if necessary in the presence of Ruis acid catalyst such as p－toluene sulfonic acid, at the temperature range from room temperature to reflux temperature.

(2)

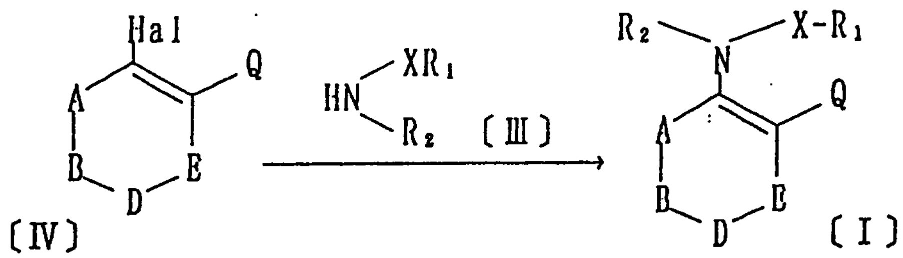

wherein $R_1$, $R_2$ X, A, B, D, E and Q represent the same meaning as described above. In the formula [IV], Hal represents halogen atom.

The compound represented by the formula [I] can be prepared by reacting the compound represented by the formula [IV] with amine represented by the formula [III] without solvent or in an inactive solvent such as benzene, toluene, DMF, DME, THF or N－methylpyrrolidone, and if necessary in the presence of base such as triethylamine, pyridine or DBU, at the temperature range from room temperature to reflux temperature.

(3)

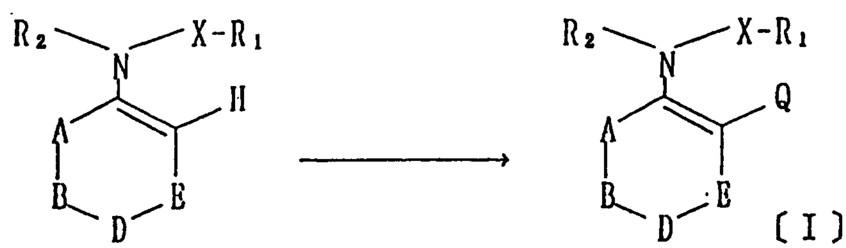

The compound represented by the formula [I] wherein Q is unsubstituted or substituted alkyl, alkenyl, alkynyl, aryl, nitro, halogen atom, $COR_4$, $CO_2R_4$ or $S(O)mR_4$ (m and $R_4$ represent the same meaning as described above) can be prepared from the compound represented by the formula [I] wherein Q is hydrogen atom.

(1) The compound represented by the formula [I] wherein Q is unsubstituted or substituted alkyl, alkenyl, alkynyl or aryl can be prepared by reacting the compound represented by the formula [I] wherein Q is hydrogen atom with alkylating agent such as alkyl halide (QHal) without solvent or in an appropriate solvent, and if necessary in the presence of base, at the temperature range from room temperature to reflux temperature.

(2) The compound represented by the formula [I] wherein Q is nitro can be prepared by nitration of the compound represented by the formula [I] wherein Q is hydrogen atom. As nitrating agent, nitric acid, fuming nitric acid, acetyl nitrate, nitroniumtetrafluoborate and the like can be used.

(3) The compound represented by the formula [I] wherein Q is halogen atom can be prepared by reacting the compound represented by the formula [I] wherein Q is hydrogen atom with a halogenizing agent such as N－chlorosuccinimide, phosphate oxychloride, sulfonyl chloride, thionyl chloride, chlorine, bromine, N－bromosuccinimide, pyridinium hydrobromide perbromide, phenyltrimethylammonium tribromide, tetrafluorosulfur, fluorine, diethylaminotrisulfur trichloride (DAST), N－fluoropyridinium triflate and the like without solvent or in an inactive solvent such as chloroform, dichloromethane, carbon tetrachloride, benzene, toluene and the like at the temperature range from room temperature to reflux

temperature.

(4) The compound represented by the formula [I] wherein Q is $COR_4$, $CO_2R_4$ or $S(O)mR_4$ can be prepared by the acylation or the sulfonation of the compound represented by the formula [I] wherein Q is hydrogen atom. These reactions proceed smoothly without solvent or in an inactive solvent such as benzene, toluene, THF DME, chloroform, dichloromethane, ether, DMF and the like, and if necessary in the presence of base such as sodium hydroxide, potassium hydroxide, triethylamine, pyridine, DBU, alcoholate and the like, at the temperature range from room temperature to reflux temperature, The acylating agents and sulfonating agents which can be used are acid chlorides such as acetyl chloride and benzoyl chloride; acid anhydrides such as acetic anhydride, trifluoroacetic anhydride and benzoic acid anhydride; chloride sulfonates such as methanesulfonyl chloride and p－toluenesulfonyl chloride; and sulfonic acid anhydrides such as methanesulfonic acid anhydride and p－toluenesulfonic acid anhydride.

(4) 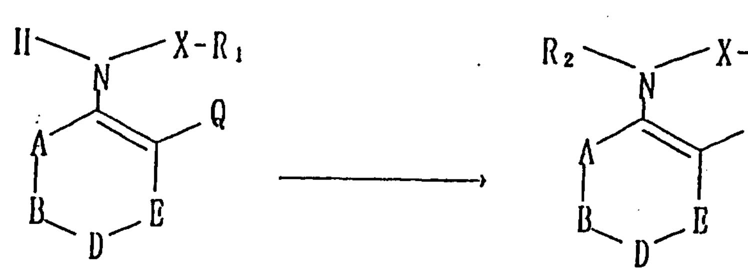

[I]

The compound represented by the formula [I] wherein $R_2$ is not hydrogen atom can be prepared from the compound represented by the formula [I] wherein $R_2$ is hydrogen atom. The reaction can be completed without solvent or in an inactive solvent such as benzene, toluene, chloroform, THF, ether, alcohol, and acetonitrile, and if necessary in the presence of base such as triethylamine, pyridine, DBU, alcoholate, and sodium hydride, at the temperature range from room temperature to reflux temperature.

After the ending of reaction, the compound represented by the formula [I] can be obtained by normal after－process. Identification of chemical structure was performed based on NMR and mass spectrum.

## Best Mode for Carrying Out the Invention

Now, the illustrative construction of the present invention is described in more detail with reference to the Examples of the present invention.

### Example 1

1－(2－chloro－5－pyridylmethylamino)－1－cyclohexene－3－on (Compound No. 1).

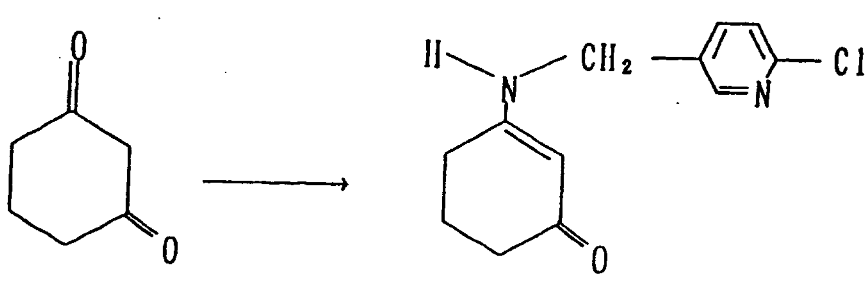

1.0 g (8.9 mmol) 1,3－cyclohexanedion was suspended in 10 ml toluene and then 1.27 g (8.9 mmol) 2－chloro－5－pyridylmethylamino and 2.0 mg p－toluenesulfonic monohydrate were added thereto, and resulting mixture was refluxed for 2 hours under heating. The reaction solution was naturally cooled at room temperature, then the separated－crystals were filtrated, obtaining 2.11 g objective compound (yield, quantitative). The melting point was 164－166 C.

Example 2

4 – (N – methyl – 2 – chloro – 5 – pyridylmethylamino) – 2,5 – dihydrofuran – 2 – on (Compound No. 2)

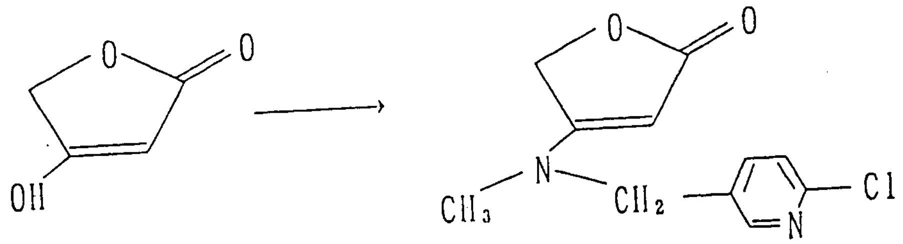

1.0 g (10.0 mmol) tetronic acid was suspended in 10 ml toluene and 1.6 g (10.2 mmol) N – methyl – 2 – chloro – 5 – pyridylmethylamine was added thereto, then the resulting mixture was refluxed for 5 hours under heating. After removing materials having low boiling point at reduced pressure from the reaction solution, 1.2 g (4 – (N – methyl – 2 – chloro – 5 pyridylmethylamino) – 2,5 – dihydrofuran – 2 – on was obtained (yield 50%) by purification using silicagel chromatography. The melting point was 105 – 107 C.

Example 3

4 – (2 – chloro – 5 – pyridylmethylamino) – 6 – methylpyran – 2 – on (Compound No. 77)

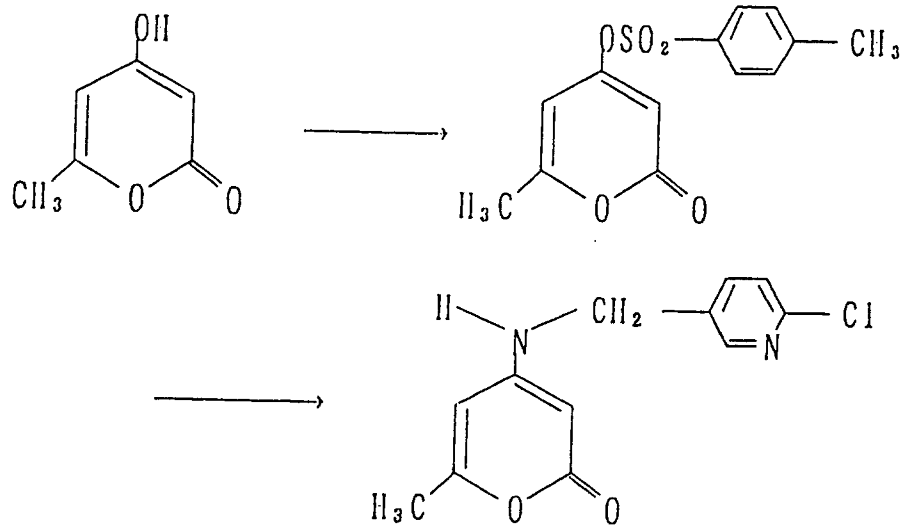

2.0 g (15.9 mmol) 4 – hydroxy – 6 – methylpyran – 2 – on was dissolved into 20 ml DMF, and 0.7 g (17.2 mmol) sodium hydride(60%) was gradually added thereto under cooling with ice. After stirring for an hour, 3 g p – toluenesulfonyl chloride was further added to the above mixture and stirred for another one hour at room temperature. The reaction mixture was poured into 100 ml ice water, then extracted with 50 ml ethyl acetate. After drying extract with magnesium sulfate anhydride, the solvents being contained are distillated at reduced pressure, then 3.8 g of substantially pure 6 – methyl – 4 – (p – toluenesulfonyloxy)pyran – 2 – onwas obtained. 1.0 g (3.6 mmol) of this compound was dissolved into 10 ml DMF, and 0.5 g (3.5 mmol) 2 – chloro – 5 – pyridylmethylamine was further added to this solution, the mixture was stirred for one night at room temperature. The reaction solution was poured into 50 ml water, and extracted with 30 ml ethyl acetate, then the resulting residues are separated and purified by using silicagel column. Thereafter. 0.30 g 4 – (2 – chloro – 5 – pyridylmethylamino) – 6 – methylpyran – 2 – on which is an objective product is obtained (yield 45%). The melting point of this product was 173 – 175 C.

Example 4

4 - (2 - chloro - 5 - pyridylmethylamino) - 1,3 - dimethyluracil (Compound No. 70)

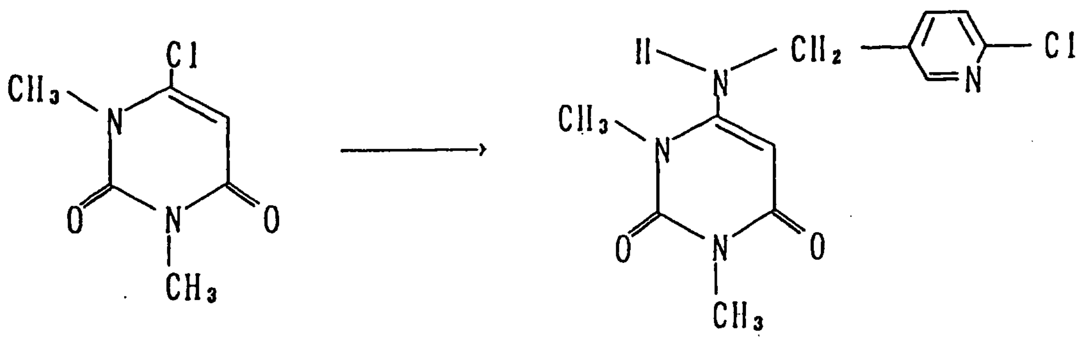

1.3 g (7.4 mmol) 4 - chloro - 1,3 - dimethyluracil was dissolved into 10 ml N - methylpyrolidone, and equivalent mol of both triethylamine and 2 - chloro - 5 - pyridylmethylamine were added thereto, then the mixture was stirred for 3 hours at 100 C. After naturally cooling the mixture to room temperature, the reaction solution was poured into 100 ml water, extracted twice with 30 ml ethyl acetate. Then the organic layer was dried with magnesium sulfate anhydride and the solvent remained were distillated at reduced pressure. The residues was separated with silicagel column chromatography and 1.0 g 4 - (2 - chloro - 5 - pyridylmethylamino - ) - 1,3 - dimethyluracil being desired is obtained. The melting point of this product was 122 - 123 C.

Example 5

4 - (2 - chloro - 5 - pyridylmethylamino) - 1,3 - dimethyl - 5 - nitrouracil (Compound No. 71)

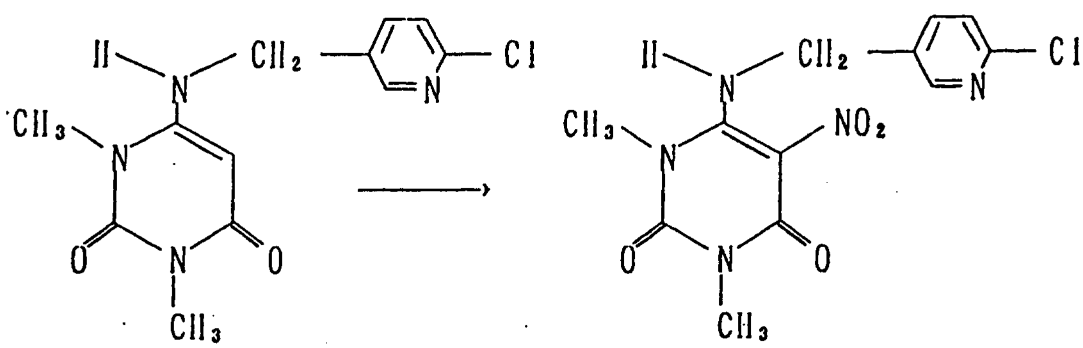

0.78 g 4 - (2 - chloro - 5 - pyridylmethylamino) - 1,3 - dimethyluracil was suspended in 30 ml concentrated sulfuric acid and 0.17 g concentrated nitric acid (d = 1.40) was fed dropwise thereto under cooling with ice. After stirring for two hours at 0 C and for one hour at room temperature, the mixture was poured into 100 ml ice water and extracted the solution twice with respective 30 ml ethyl acetate. Then the organic layer was washed with sodium hydrogencarbonate solution and saturated sodium chloride solution and dried with magnesium sulfate anhydride. Then solvents are distillated at reduced pressure and obtained crude product. This crude product was processed with mixed - solvent of chloroform - n - hexane, them obtained 0.72 g of desired product (yield 80%). The melting point of this product was 147 - 148 C.

The typical examples of the compounds of this invention are illustrated in Table 1 as well as the Examples described above.

Table 1

| Compound No. | Structure Formula 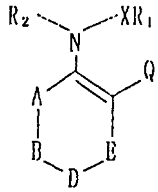 | | | | | Pysical Properties [ ]m.p. ℃ |
|---|---|---|---|---|---|---|
| | $R_1$ X | $R_2$ | −A−B−D− | E | Q | |
| 1 | 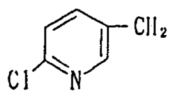 | H | $-CH_2-CH_2-CH_2-$ | 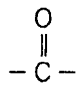 | H | [164-166] |
| 2 | 〃 | $CH_3$ | 〃 | 〃 | 〃 | [ 86-88 ] |
| 3 | 〃 | $C_2H_5$ | 〃 | 〃 | 〃 | |
| 4 | 〃 | $C_3H_7$(n) | 〃 | 〃 | 〃 | |
| 5 | 〃 | $C_3H_7$(is) | 〃 | 〃 | 〃 | |
| 6 | 〃 | 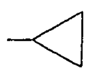 | 〃 | 〃 | 〃 | |
| 7 | 〃 | 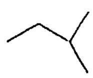 | 〃 | 〃 | 〃 | $n_D^{25}$ 1.6005 |
| 8 | 〃 | $-CH_2-$ 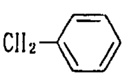 | 〃 | 〃 | 〃 | |
| 9 | 〃 | $CH_2CH{=}CH_2$ | 〃 | 〃 | 〃 | |
| 10 | 〃 | $CH_2C{\equiv}CH$ | 〃 | 〃 | 〃 | |

| | | | | | | |
|---|---|---|---|---|---|---|
| 11 | 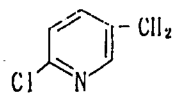 | $OCH_3$ | $-CH_2-CH_2-CH_2-$ | 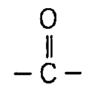 | H | [101-104] |
| 12 | 〃 | $COCH_3$ | 〃 | 〃 | 〃 | |
| 13 | 〃 | $COCF_3$ | 〃 | 〃 | 〃 | $n_D^{25}$ 1.5303 |
| 14 | 〃 | CO 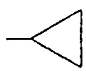 | 〃 | 〃 | 〃 | |
| 15 | 〃 | $SO_2CH_3$ | 〃 | 〃 | 〃 | |
| 16 | 〃 | 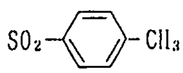 | 〃 | 〃 | 〃 | |
| 17 | 〃 | H | 〃 | 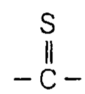 | 〃 | [130-134dec] |
| 18 | 〃 | $CH_3$ | 〃 | 〃 | 〃 | |
| 19 | 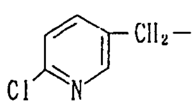 | H | 〃 | 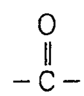 | $CH_3$ | [157-158] |
| 20 | 〃 | 〃 | 〃 | 〃 | ※1 | |

※1 $CH_2CH=CH_2$

| | | | | | | |
|---|---|---|---|---|---|---|
| 21 | 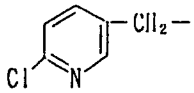 | H | $-CH_2CH_2CH_2-$ | $-\overset{O}{\overset{\|}{C}}-$ | ※2 | |
| 22 | 〃 | 〃 | 〃 | 〃 | 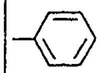 | |
| 23 | 〃 | 〃 | 〃 | 〃 | Cl | [160-161] |
| 24 | 〃 | 〃 | 〃 | 〃 | $NO_2$ | $n_D^{25}$ 1.5908 |
| 25 | 〃 | 〃 | 〃 | 〃 | CN | |
| 26 | 〃 | 〃 | 〃 | 〃 | $COCH_3$ | |
| 27 | 〃 | 〃 | 〃 | 〃 | $COCF_3$ | $n_D^{25}$ 1.5225 |
| 28 | 〃 | 〃 | 〃 | 〃 | $SO_2CH_3$ | |
| 29 | 〃 | 〃 | $-CH_2CH_2\overset{CH_3}{\overset{\|}{CH}}-$ | 〃 | H | [200-201] |
| 30 | 〃 | 〃 | $-CH_2CH_2\underset{CH_3}{\underset{\|}{\overset{CH_3}{\overset{\|}{C}}}}-$ | 〃 | 〃 | [193-195] |

※2 $CH_2C \equiv CH$

| | | | | | | |
|---|---|---|---|---|---|---|
| 31 | 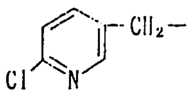 | H | SCH3<br>\|<br>$-CH_2CH_2-CH-$ | O<br>‖<br>$-C-$ | H | [180-182]dec |
| 32 | 〃 | 〃 | $CH_2CH_2CN$<br>\|<br>$-CH_2CH_2CH-$ | 〃 | 〃 | [176-177] |
| 33 | 〃 | 〃 | 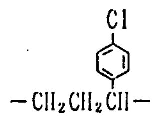 | 〃 | 〃 | [241-243] |
| 34 | 〃 | 〃 | $CH_3$<br>\|<br>$-CH_2CHCH_2-$ | 〃 | 〃 | [176-177] |
| 35 | 〃 | 〃 | $CH_3$<br>$CH_2CH$<br>\| $SC_2H_5$<br>$-CH_2CHCH_2-$ | 〃 | 〃 | [137-138] |
| 36 | 〃 | 〃 | 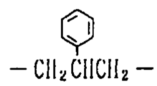 | 〃 | 〃 | [216-127] |
| 37 | 〃 | 〃 | 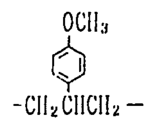 | 〃 | 〃 | [210-211] |
| 38 | 〃 | 〃 | 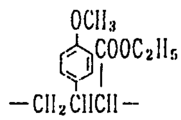 | 〃 | 〃 | [224-226] |
| 39 | 〃 | 〃 | $CH_3$<br>\|<br>$-CH_2CCH_2-$<br>\|<br>$CH_3$ | 〃 | 〃 | [199-201] |
| 40 | 〃 | 〃 | 〃 | 〃 | $NO_2$ | [181-183] |

| | | | | | | |
|---|---|---|---|---|---|---|
| 41 | 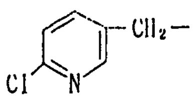 | H | 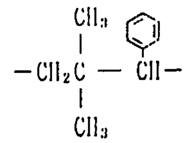 | $-\overset{O}{\overset{\|}{C}}-$ | H | [182-183] |
| 42 | 〃 | $CH_3$ | $-CH_2CH_2\overset{SCH_3}{\overset{\|}{C}}H-$ | 〃 | 〃 | [108-110] |
| 43 | 〃 | 〃 | $-CH_2\overset{CH_3}{\overset{\|}{C}}HCH_2-$ | 〃 | 〃 | $n_D^{25}$ 1.5992 |
| 44 | 〃 | H | $-CH_2\overset{CH_2OCH_3}{\overset{\|}{C}}HCH_2-$ | 〃 | 〃 | [139-140] |
| 45 | 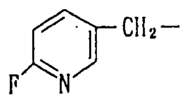 | 〃 | $-CH_2CH_2CH_2-$ | 〃 | 〃 | |
| 46 | 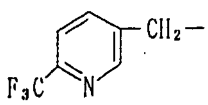 | 〃 | 〃 | 〃 | 〃 | |
| 47 | 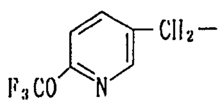 | 〃 | 〃 | 〃 | 〃 | |
| 48 | 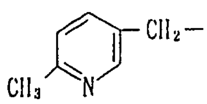 | 〃 | 〃 | 〃 | 〃 | |
| 49 | 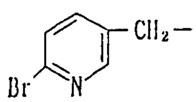 | 〃 | 〃 | 〃 | 〃 | |
| 50 | 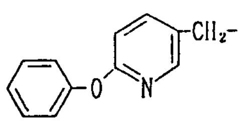 | 〃 | 〃 | 〃 | 〃 | |

| | | | | | | |
|---|---|---|---|---|---|---|
| 51 | 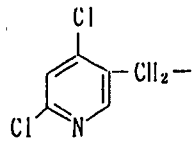 | H | $-CH_2CH_2CH_2-$ | $-\overset{O}{\overset{\|\|}{C}}-$ | H | |
| 52 | 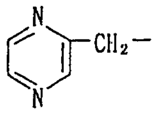 | ″ | ″ | ″ | ″ | |
| 53 | 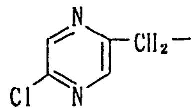 | ″ | ″ | ″ | ″ | |
| 54 | 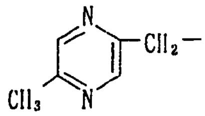 | ″ | ″ | ″ | ″ | |
| 55 | 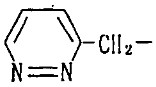 | ″ | ″ | ″ | ″ | |
| 56 | 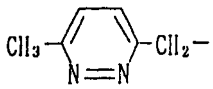 | ″ | ″ | ″ | ″ | |
| 57 | 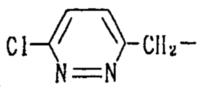 | ″ | ″ | ″ | ″ | |
| 58 | 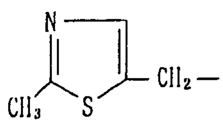 | ″ | ″ | ″ | ″ | |
| 59 | 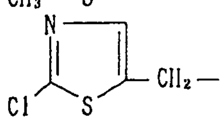 | ″ | ″ | ″ | ″ | [144-146] |
| 60 | ″ | $CH_3$ | ″ | ″ | ″ | $n_D^{25}$ 1.6208 |

| | | | | | | |
|---|---|---|---|---|---|---|
| 61 | 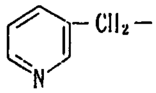 | H | $-CH_2CH_2CH_2-$ | O<br>‖<br>$-C-$ | H | [ 73-76 ] |
| 62 | 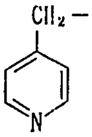 | 〃 | 〃 | 〃 | 〃 | |
| 63 | 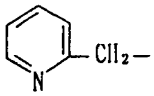 | 〃 | 〃 | 〃 | 〃 | |
| 64 | 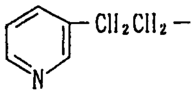 | 〃 | 〃 | 〃 | 〃 | |
| 65 | 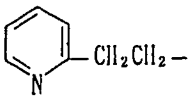 | 〃 | 〃 | 〃 | 〃 | |
| 66 | 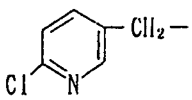 | 〃 | $-CH_2CH_2NH-$ | 〃 | 〃 | [191-192] |
| 67 | 〃 | 〃 | $CH_3$<br>$-CH_2CH_2N-$ | 〃 | 〃 | |
| 68 | 〃 | 〃 | 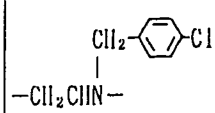 | 〃 | 〃 | [154-156] |
| 69 | 〃 | 〃 | $CH_3$<br>$\vert\ \ CH_3$<br>$-CH=C-N-$ | 〃 | $NO_2$ | $n_D^{24.5}$ 1.4972 |
| 70 | 〃 | 〃 | O<br>$CH_3$ ‖ $CH_3$<br>$-N\ -C-N-$ | 〃 | H | [122-123] |

| | | | | | | |
|---|---|---|---|---|---|---|
| 71 | 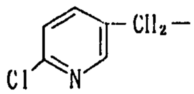 | H | O<br>$CH_3$ ‖ $CH_3$<br>$-N-C-N-$ | O<br>‖<br>$-C-$ | $NO_2$ | [147-148] |
| 72 | 〃 | 〃 | $CH_3$ $CH_3$<br>$-N-SO_2-N-$ | 〃 | H | [159-160] |
| 73 | 〃 | 〃 | 〃 | 〃 | $NO_2$ | [210-211] |
| 74 | 〃 | 〃 | $-CH_2CH_2O-$ | 〃 | H | [189-190] |
| 75 | 〃 | 〃 | $CH_3$<br>\|<br>$-CH_2CHO-$ | 〃 | 〃 | [143-144] |
| 76 | 〃 | 〃 | 〃 | 〃 | $NO_2$ | [152-154] |
| 77 | 〃 | 〃 | $CH_3$<br>\|<br>$-CH=C-O-$ | 〃 | H | [173-175] |
| 78 | 〃 | 〃 | $CH_3$<br>\|<br>$-CH_2C-S-$<br>\|<br>$CH_3$ | 〃 | 〃 | [205-207] |
| 79 | 〃 | 〃 | 〃 | S<br>‖<br>$-C-$ | 〃 | [153-155]dec |
| 80 | 〃 | 〃 | $-CH_2SCH_2-$ | O<br>‖<br>$-C-$ | 〃 | [217-218] |

| | | | | | | |
|---|---|---|---|---|---|---|
| 81 | 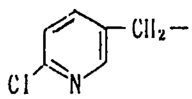 | H | $-CH_2SC(CH_3)_2-$ | $-C(=O)-$ | H | [196-197] |
| 82 | 〃 | $CH_3$ | $-CH_2CH_2O-$ | 〃 | 〃 | [viscous oil] |
| 83 | 〃 | H | $-CH_2O-$ | 〃 | 〃 | [136-138] |
| 84 | 〃 | $CH_3$ | 〃 | 〃 | 〃 | [105-107] |
| 85 | 〃 | $C_2H_5$ | 〃 | 〃 | 〃 | [103-104] |
| 86 | 〃 | $C_3H_7(n)$ | 〃 | 〃 | 〃 | [ 97-100] |
| 87 | 〃 | $C_3H_7(is)$ | 〃 | 〃 | 〃 | |
| 88 | 〃 | 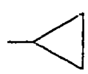 | 〃 | 〃 | 〃 | [ 89-92 ] |
| 89 | 〃 | 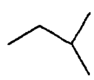 | 〃 | 〃 | 〃 | $n_D^{25}$ 1.5725 |
| 90 | 〃 | 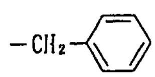 | 〃 | 〃 | 〃 | |

| | | | | | | |
|---|---|---|---|---|---|---|
| 91 | 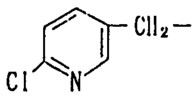 | $CH_2CH=CH_2$ | $-CH_2O-$ | $-\overset{O}{\overset{\|}{C}}-$ | H | [ 78-79 ] |
| 92 | 〃 | $CH_2C≡CH$ | 〃 | 〃 | 〃 | |
| 93 | 〃 | $CONHCH_3$ | 〃 | 〃 | 〃 | [183-191] |
| 94 | 〃 | $COCH_3$ | 〃 | 〃 | 〃 | |
| 95 | 〃 | $COCF_3$ | 〃 | 〃 | 〃 | |
| 96 | 〃 | CO 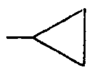 | 〃 | 〃 | 〃 | [161-164] |
| 97 | 〃 | $SO_2CH_3$ | 〃 | 〃 | 〃 | |
| 98 | 〃 | $OCH_3$ | 〃 | 〃 | 〃 | [128-129] |
| 99 | 〃 | H | 〃 | $-\overset{S}{\overset{\|}{C}}-$ | 〃 | |
| 100 | 〃 | $CH_3$ | 〃 | 〃 | 〃 | |

| | | | | | | |
|---|---|---|---|---|---|---|
| 101 | 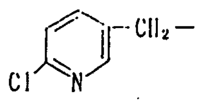 | H | $-CH_2O-$ | 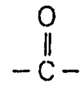 | $CH_3$ | |
| 102 | 〃 | 〃 | 〃 | 〃 | Cl | |
| 103 | 〃 | 〃 | 〃 | 〃 | Br | [144-145] |
| 104 | 〃 | $CH_3$ | 〃 | 〃 | F | [100-103] |
| 105 | 〃 | 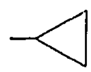 | 〃 | 〃 | 〃 | [ 81-83 ] |
| 106 | 〃 | H | 〃 | 〃 | $NO_2$ | [218-220] |
| 107 | 〃 | 〃 | 〃 | 〃 | CN | |
| 108 | 〃 | 〃 | 〃 | 〃 | $SO_2CH_3$ | [191-192] |
| 109 | 〃 | 〃 | 〃 | 〃 | $COCH_3$ | |
| 110 | 〃 | 〃 | 〃 | 〃 | $COCF_3$ | [173-176dec] |

| | | | | | | |
|---|---|---|---|---|---|---|
| 111 | 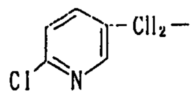 | H | $-CH_2S-$ | $-\overset{O}{\overset{\|}{C}}-$ | H | [183-184] |
| 112 | 〃 | $CH_3$ | 〃 | 〃 | 〃 | [104-105] |
| 113 | 〃 | $C_2H_5$ | 〃 | 〃 | 〃 | |
| 114 | 〃 | 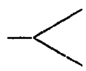 | 〃 | 〃 | 〃 | |
| 115 | 〃 | 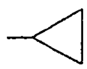 | 〃 | 〃 | 〃 | |
| 116 | 〃 | H | 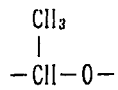 | 〃 | 〃 | [157-158] |
| 117 | 〃 | $CH_3$ | 〃 | 〃 | 〃 | $n_D^{25}$ 1.5737 |
| 118 | 〃 | 〃 | 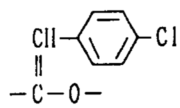 | 〃 | 〃 | [ 78-80 ] |
| 119 | 〃 | 〃 | 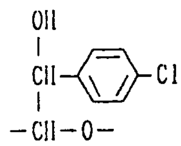 | 〃 | 〃 | [195-198] |
| 120 | 〃 | 〃 | 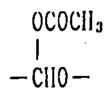 | 〃 | 〃 | [118-121] |

| | | | | | | |
|---|---|---|---|---|---|---|
| 121 | 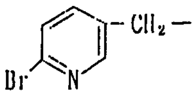 | $CH_3$ | $-CH_2O-$ | 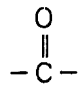 | H | |
| 122 | 〃 | 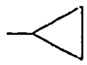 | 〃 | 〃 | 〃 | |
| 123 | 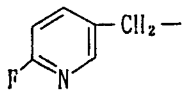 | $CH_3$ | 〃 | 〃 | 〃 | |
| 124 | 〃 | 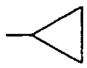 | 〃 | 〃 | 〃 | |
| 125 | 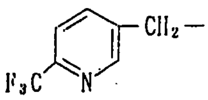 | $CH_3$ | 〃 | 〃 | 〃 | |
| 126 | 〃 | 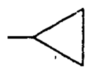 | 〃 | 〃 | 〃 | |
| 127 | 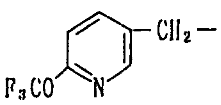 | $CH_3$ | 〃 | 〃 | 〃 | |
| 128 | 〃 | 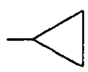 | 〃 | 〃 | 〃 | |
| 129 | 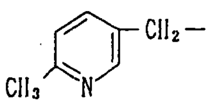 | $CH_3$ | 〃 | 〃 | 〃 | |
| 130 | 〃 | 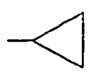 | 〃 | 〃 | 〃 | |

| | | | | | | |
|---|---|---|---|---|---|---|
| 131 | 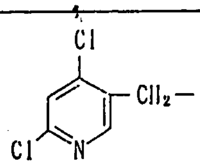 | CH₃ | $-CH_2O-$ | 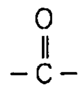 | H | |
| 132 | 〃 | 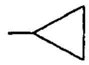 | 〃 | 〃 | 〃 | |
| 133 | 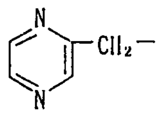 | $CH_3$ | 〃 | 〃 | 〃 | |
| 134 | 〃 | 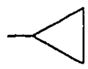 | 〃 | 〃 | 〃 | |
| 135 | 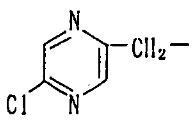 | $CH_3$ | 〃 | 〃 | 〃 | |
| 136 | 〃 | 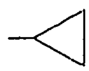 | 〃 | 〃 | 〃 | |
| 137 | 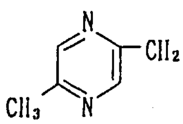 | $CH_3$ | 〃 | 〃 | 〃 | |
| 138 | 〃 | 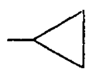 | 〃 | 〃 | 〃 | |
| 139 | 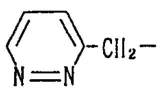 | $CH_3$ | 〃 | 〃 | 〃 | |
| 140 | 〃 | 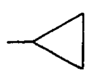 | 〃 | 〃 | 〃 | |

| | | | | | | |
|---|---|---|---|---|---|---|
| 141 | 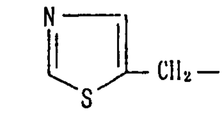 | $CH_3$ | $-CH_2O-$ | 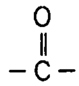 | H | |
| 142 | 〃 | 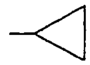 | 〃 | 〃 | 〃 | |
| 143 | 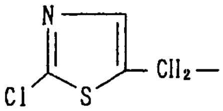 | H | 〃 | 〃 | 〃 | [143-145] |
| 144 | 〃 | $CH_3$ | 〃 | 〃 | 〃 | $n_D^{25}$ 1.6035 |
| 145 | 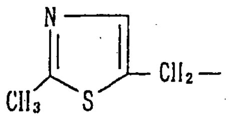 | $CH_3$ | 〃 | 〃 | 〃 | |
| 146 | 〃 | 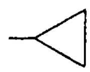 | 〃 | 〃 | 〃 | |
| 147 | 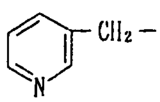 | $CH_3$ | 〃 | 〃 | 〃 | |
| 148 | 〃 | 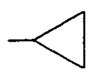 | 〃 | 〃 | 〃 | |
| 149 | 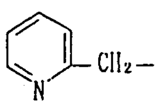 | $CH_3$ | 〃 | 〃 | 〃 | |
| 150 | 〃 | 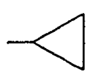 | 〃 | 〃 | 〃 | |

| | | | | | | |
|---|---|---|---|---|---|---|
| 151 | 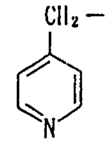 | CH₃ | $-CH_2O-$ | O<br>‖<br>−C− | H | |
| 152 | 〃 | 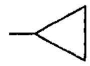 | 〃 | 〃 | 〃 | |
| 153 | 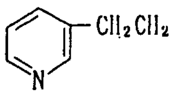 | $CH_3$ | 〃 | 〃 | 〃 | |
| 154 | 〃 | 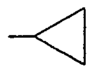 | 〃 | 〃 | 〃 | |
| 155 | 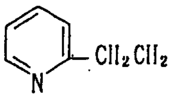 | $CH_3$ | 〃 | 〃 | 〃 | |
| 156 | 〃 | 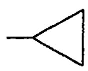 | 〃 | 〃 | 〃 | |
| 157 | 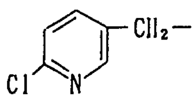 | H | $-CH_2NH-$ | 〃 | 〃 | [194-200dec] |
| 158 | 〃 | $CH_3$ | 〃 | 〃 | 〃 | [193-198dec] |
| 159 | 〃 | H | $CH_3$<br>\|<br>−C−NH−<br>\|<br>$CH_3$ | 〃 | 〃 | [214-215] |
| 160 | 〃 | $CH_3$ | 〃 | 〃 | 〃 | [147-148] |

| | | | | | | |
|---|---|---|---|---|---|---|
| 161 | 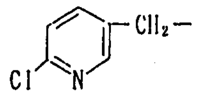 | H | 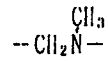 | 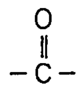 | H | [192-193dec] |
| 162 | 〃 | $CH_3$ | 〃 | 〃 | 〃 | [114-115] |
| 163 | 〃 | H | 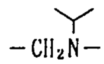 | 〃 | 〃 | [223-226dec] |
| 164 | 〃 | $CH_3$ | 〃 | 〃 | 〃 | [101-102] |
| 165 | 〃 | 〃 | 〃 | 〃 | ※3 | [133-136] |
| 166 | 〃 | $C_3H_7$(n) | $-CH_2NH-$ | 〃 | H | |
| 167 | 〃 | $C_3H_7$(is) | 〃 | 〃 | 〃 | |
| 168 | 〃 | 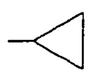 | 〃 | 〃 | 〃 | |
| 169 | 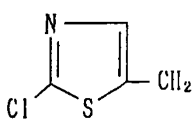 | $CH_3$ | 〃 | 〃 | 〃 | |
| 170 | 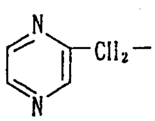 | 〃 | 〃 | 〃 | 〃 | |

※3

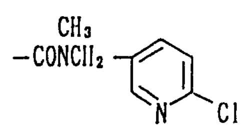

| | | | | | | |
|---|---|---|---|---|---|---|
| 171 | 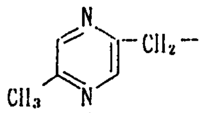 | $CH_3$ | $-CH_2NH-$ | 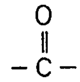 | H | |
| 172 | 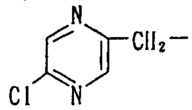 | 〃 | 〃 | 〃 | 〃 | |
| 173 | 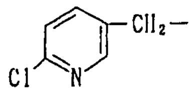 | 〃 | 〃 | 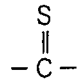 | 〃 | |
| 174 | 〃 | H | $-CH_2CH_2-$ | 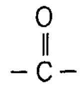 | 〃 | [173-175] |
| 175 | 〃 | $CH_3$ | 〃 | 〃 | 〃 | $n_D^{25.5}$ 1.6092 |
| 176 | 〃 | $C_2H_5$ | 〃 | 〃 | 〃 | |
| 177 | 〃 | $C_3H_7(n)$ | 〃 | 〃 | 〃 | |
| 178 | 〃 | $C_3H_7(is)$ | 〃 | 〃 | 〃 | |
| 179 | 〃 | 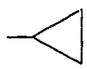 | 〃 | 〃 | 〃 | |
| 180 | 〃 | $COCH_3$ | 〃 | 〃 | 〃 | |

| | | | | | | |
|---|---|---|---|---|---|---|
| 181 | 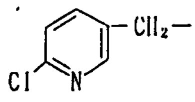 | $SO_2CH_3$ | $-CH_2CH_2-$ | 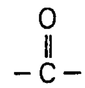 | H | |
| 182 | // | $CH_3$ | // | 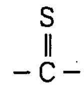 | // | |
| 183 | 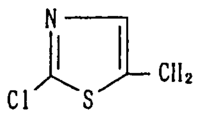 | // | // | 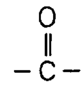 | // | |
| 184 | 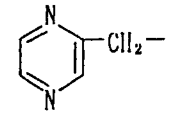 | // | // | // | // | |
| 185 | 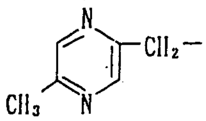 | // | // | // | // | |
| 186 | 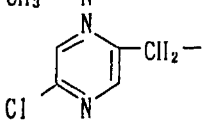 | // | // | // | // | |
| 187 | 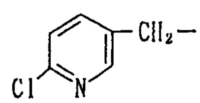 | // | // | // | $CH_3$ | [174-175] |
| 188 | // | H | // | // | $NO_2$ | [165-167] |

The compound specified in the present invention demonstrates excellent insecticidal activity against various pest insects such as armyworm, diamond back moth, aphids, planthoppers, and brown leafhoppers. Recently, some of the pest insects have been developing resistance against insecticides such as organophosphorus compounds, and carbamate compounds, whereby the deficiency of insecticidal efficacy of those insecticides are coming up to serious problem. Because of this reason, novel insecticides being effective even against those resistant strain of pest insects are urgently required. The compound specified in the present invention has an excellent insecticidal efficacy not only against the insecticides-sensitive pest insects but also against the resistant strain of pest insect for organophosphorus compounds and carbamate compounds.

The insecticide specified in the present invention contains the compound represented by the general formula [I] as an active ingredient. Although this compound can be used without processing, it is normally used in the typical form for agricultural chemicals such as wettable powder, water soluble powder, dust formulation, emulsifiable concentrate, granular formulation, and flowable formulation. As an additive and a carrier, when using them for solid formulation, plant-origin powder such as soybean powder and wheat flour; mineral fine powder such as diatomaceous earth, apatite, gypsum, talc, bentonite, and clay; and organic and inorganic compounds such as sodium benzoate, urea and Glauber' s salt can be used.

In the case of liquid formulation, vegetable oils, mineral oils, distillate fractions of petroleum such as kerosene, xylene and solvent naphtha, and cyclohexane, cyclohexanone, dimethylformamide dimethylsulfoxide, trichloroethylene, methyl isobutyl ketone and water can be used as a solvent. In order to keep homogeneous and stable dilution, if necessary, surface active agents are allowable to be added. Thus prepared wettable powder, emulsifiable concentrate, water soluble powder and flowable are diluted to an indicated concentration as suspension, emulsion in water or aqueous solution. Then, they are sprayed, but dust and granular are applied without any dilution, for crop plants.

Although the compounds in the invention are, of course, sufficiently effective even in single use, they are also allowable to be blended with many kinds of insecticides, acaricides and fungicides.

Insecticides, acaricides and fungicides with which the invented compounds are allowable to be blended are shown as follows:

Acaricides (Fungicide):

chlorobenzilate, chloropropilate, proclonol, phenisobromolate, dicofol, dinobuton, binapacryl, chlorophenamidin, amitoraz, BPPS, PPPS, benzomate hexythioazox, phenbutatin oxide, polynactin, quinomethionate, thioquinox, CPCBS, tetradifon, kayacide, avermectin, calcium polysulfide, clofentezin, fulbenzimin, fulfenoxuron, BCPE, cyhexatin, pyridaben fenpyroximate, thiophanate methyl, benomyl, thiuram, IBP, EDDP, fthalide, probenazole, isoprothiolane, TPN, captan, polyoxin, blastcidin − S, kasugamycin, validamycin, tricyclazole, pyroquilon, phenazin oxide, mepronil, fultoranil, pencycuron, iprodione, hymexazol, metalaxil, triflumizol, diclomezin and tecloftalam;

Organophosphate and carbamate insecticides (Acaricides):

fenthion, fenitrothion, diazinon, chlorpyriphos, ESP, vamidothion, fenthoate, dimethoate, formothion, malathion, dipterex, thiometon, phosmet, menazon, dichlorvos, acephate, EPBP, diaryfol, methyl paration, oxydimethon methyl, ethion, pyrachlofos, monocrotophos, aldicarb, propoxur, BPMC, MTMC, NAC cartap, carbosulfan, benfuracarb, pyrimicarb, ethofencarb, phenoxycarb and thiodicarb;

Pyrethroids insecticides (Acaricides):

permethrin, cypermethrin, decamethrin, fenvalerate, fenpropathrin, pyrethrin, allethrin, tetramethrin, resmethrin, dimethrin, propathrin, biffenthrin, prothrin, fluvalinate, cyfluthrin, cyhalothrin, flucythrinate, ethofenprox, cycloprothrin, tralomethrin, silaneophne;

Benzoylphenylureas and other insecticides:

diflubenzuron, chlofluazuron, triflumuron, teflubenzuron, buprofezin and machine oil.

The examples of the formulations are shown as follows, however, the scope of the present invention shall not be limited by these descriptions.

Example 6 Emulsifiable concentrate

| | |
|---|---|
| The invented compound | 10 parts |
| alkylphenylpolyoxyethylene | 5 parts |
| dimethylformamide | 50 parts |
| xylene | 35 parts |

These are mixed to be a solution and sprayed by dilution with water when it is used.

Example 7 Wettable powder

| | |
|---|---|
| The invented compound | 20 parts |
| sulfate ester of higher alcohol | 5 parts |
| diatomaceous earth | 70 parts |
| artificial silicate | 5 parts |

These are mixed, micronized to be fine powder and sprayed by dilution with water when it is used.

Example 8 Dust

| | |
|---|---|
| The invented compound | 5 parts |
| talc | 94.7 parts |
| artificial silicate | 0.3 parts |

These are mixed, pulvalized to be a dusting powder and sprayed without dilution when it is used.

Example 9 Granular formulation

| | |
|---|---|
| The invented compound | 5 parts |
| clay | 73 parts |
| bentonite | 20 parts |
| sodium dioctylsulfosuccinate | 1 parts |
| sodium phosphate | 1 parts |

These are prepared to be granules and applied without dilution when it is used.

Possibility for Industrial Use

Test Example 1: Efficacy against cotton aphids

Cotton aphids were inoculated at the rate of 30 – 50 aphids per plot on 10 days old cucumber leaves grown in 10 cm pot by using small brush respectively. The aphids injured during inoculation were eliminated after one day, then the compound solution adjusted to 125 ppm concentration by dilution emulsifiable concentrate according to the recipe of the Example 6 with water was sprayed thereto. The aphids were allowed to stand in incubator maintained at 25°C and 65% R.H., then counted the number of living aphids after 7 days whereby the control ratio was determined in comparison with the untreated plot. The results are summarized in Table 2.

Table 2

| Compound No. | Control Efficacy (7 days later) 125 ppm |
|---|---|
| 1 | 100 % |
| 2 | 100 |
| 7 | 100 |
| 11 | 100 |
| 13 | 100 |
| 17 | 100 |
| 27 | 100 |
| 29 | 100 |
| 31 | 100 |
| 59 | 100 |
| 60 | 100 |
| 61 | 100 |
| 74 | 100 |
| 75 | 100 |
| 82 | 100 |
| 83 | 100 |
| 84 | 100 |
| 85 | 100 |
| 86 | 100 |
| 88 | 100 |
| 89 | 100 |

to be continued

| | |
|---|---|
| 91 | 100% |
| 98 | 100 |
| 103 | 100 |
| 104 | 100 |
| 105 | 100 |
| 111 | 100 |
| 112 | 100 |
| 116 | 100 |
| 117 | 100 |
| 119 | 100 |
| 143 | 100 |
| 144 | 100 |
| 157 | 100 |
| 158 | 100 |
| 175 | 100 |
| 188 | 100 |
| Comparative Compound A | 0 |

Comparative Compound A :

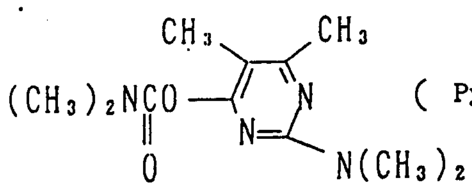

( Pyrimicarb )

Test Example 2: Efficacy against planthoppers

Young seedlings of rice plant at the stage of 7 days after germination were dipped for 30 seconds in the compound solution of the emulsifiable concentrate according to the recipe of Example 6 adjusted to 125 ppm concentration by diluting with water. After seedlings were dried, they were put into the inside of test tubes and 10 third instar larva of resistant-strain of planthoppers for organophosphate and carbamate insecticides were respectively inoculated in each test tube. The test tubes were shielded with gauze and allowed to stand in the incubator maintained at 25°C and 65% R.H., then the percentage killed were

counted after 5 days. The results were summarized in Table 3.

Table 3

| Compound No. | Mortality (5days later)<br>125 ppm |
|---|---|
| 1 | 100 % |
| 2 | 100 |
| 23 | 100 |
| 29 | 100 |
| 30 | 100 |
| 31 | 100 |
| 34 | 100 |
| 42 | 100 |
| 59 | 100 |
| 60 | 100 |
| 69 | 100 |
| 70 | 100 |
| 71 | 100 |
| 73 | 100 |
| 74 | 100 |
| 75 | 100 |
| 77 | 100 |
| 80 | 100 |
| 82 | 100 |
| 83 | 100 |
| 84 | 100 |
| 85 | 100 |

to be continued

| | |
|---|---|
| 86 | 100 % |
| 88 | 100 |
| 89 | 100 |
| 91 | 100 |
| 96 | 100 |
| 98 | 100 |
| 103 | 100 |
| 104 | 100 |
| 105 | 100 |
| 111 | 100 |
| 112 | 100 |
| 116 | 100 |
| 117 | 100 |
| 143 | 100 |
| 144 | 100 |
| 161 | 100 |
| 175 | 100 |
| 187 | 100 |
| Comparative Compound B | 0 |

Comparative Compound B :

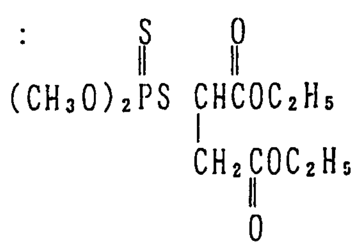

(Malathion)

## Claims

**1.** A compound represented by the general formula [I]:

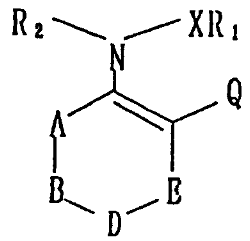

[ I ]

wherein $R_1$ represents an unsubstituted or substituted 5− or 6−membered aromatic heterocyclic radical containing nitrogen; X represents an unsubstituted or substituted alkylene or alkylidene;$R_2$ represents hydrogen, unsubstituted or substituted alkyl, alkenyl, alkynyl, cycloalkyl, cycloalkenyl, aryl or $-Y-R_3$; Y represents O, $S(O)_1$ CO or $CO_2$; 1 represents 0, 1 or 2;$R_3$ represents hydrogen, unsubstituted or substituted alkyl, alkenyl, alkynyl, cycloalkyl, cycloalkenyl or aryl group. A, B and D each represent independently an unsubstituted or substituted carbon atom, hetero atom or a single bond; E represents CO and CS; Q represents a hydrogen atom, an unsubstituted or substituted alkyl, alkenyl, alkynyl, aryl, nitro, halogen atom or $Z-R_4$; Z represents CO, $CO_2$ or S(O)m; m represents 0, 1 or 2;$R_4$ represents an unsubstituted or substituted alkyl, alkenyl, alkynyl, cycloalkyl, cycloalkenyl or aryl; and salts thereof.

**2.** a process for preparing a compound represented by the general formula [I]:

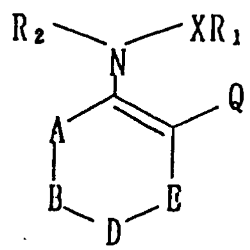

[ I ]

wherein $R_1$, $R_2$, X, A, B, D, E and Q represent the same meaning as described above; and said process is characterized by reacting a compound represented by the general formula [II]:

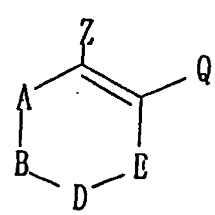

[ II ]

wherein Z represents S(O)n $R_5$, $OR_6$ or an enolic hydroxy group; n represents 0, 1 or 2; $R_5$ represents an unsubstituted or substituted alkyl having 1 to 4 carbon atoms or phenyl; R6 represents an unsubstituted or substituted alkyl having 1 to 4 carbon atoms, phenyl or $SO_2R_7$; $R_7$ represents an unsubstituted or substituted alkyl having 1 to 4 carbon atoms or phenyl; and A, B, D, E and Q represent the same meaning as described above; with a compound represented by the general formula [III]:

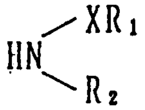

[ III ]

wherein $R_1$, $R_2$ and X represent the same meaning as described above.

3. A process for preparing a compound represented by the general formula [I]:

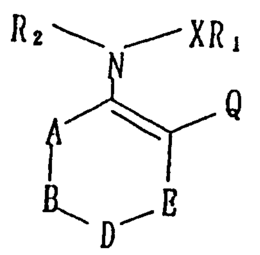

[ I ]

wherein $R_1$, $R_2$ X, A, B, D, E and Q represent the same meaning as described above, and said process is characterized by reacting a compound represented by the formula [IV]:

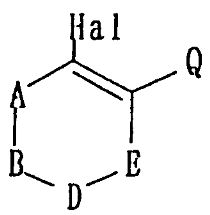

[IV]

wherein Hal represents a halogen atom; A, B, D, E and Q represent the same meaning as described above, with a compound represented by the formula [III]:

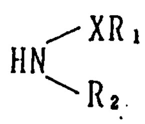

[III]

wherein $R_1$, $R_2$, X, A, B, D, E and Q represent the same meaning as described above.

4. An insecticide characterized by containing one or more compounds represented by the general formula [I] as active ingredient:

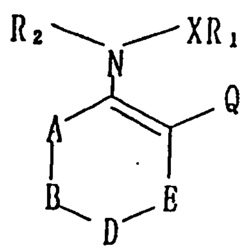

[ I ]

wherein $R_1$, $R_2$, X, A, B, D, E and Q represent the same meaning as described above.

# INTERNATIONAL SEARCH REPORT

International Application No PCT/JP91/00889

**I. CLASSIFICATION OF SUBJECT MATTER** (if several classification symbols apply, indicate all)[6]

According to International Patent Classification (IPC) or to both National Classification and IPC

Int. $Cl^5$ C07D213/61, C07D213/64, C07D237/08, C07D237/12, C07D277/28, C07D277/32, C07D401/12, C07D403/12,

**II. FIELDS SEARCHED**

Minimum Documentation Searched[7]

| Classification System | Classification Symbols |
|---|---|
| IPC | C07D213/61, C07D213/64, C07D237/08, C07D237/12, C07D277/28, C07D277/32, C07D401/12, C07D403/12, C07D405/12, C07D409/12, C07D417/12 |

Documentation Searched other than Minimum Documentation to the Extent that such Documents are Included in the Fields Searched[8]

**III. DOCUMENTS CONSIDERED TO BE RELEVANT**[9]

| Category * | Citation of Document, [11] with indication, where appropriate, of the relevant passages [12] | Relevant to Claim No. [13] |
|---|---|---|
| Y | JP, A, 59-116241 (USV Pharmaceutical Corp.), July 5, 1984 (05. 07. 84), (Family: none) | 1-4 |
| Y | JP, A, 50-95277 (The Upjohn Co.), July 29, 1975 (29. 07. 75), (Family: none) | 1-4 |
| Y | JP, A, 55-45695 (Bayer AG), March 31, 1980 (31. 03. 80), & EP, A1, 9693 | 1-4 |
| Y | JP, A, 57-77677 (Smithkline Corp.), May 15, 1982 (15. 05. 82), & US, A, 4340733 & EP, A2, 47164 & DK, A, 325981 | 1-4 |
| Y | JP, A, 56-57783 (K.K. Otsuka Seiyaku Kojyo), May 20, 1981 (20. 05. 81), (Family: none) | 1-4 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| September 10, 1991 (10. 09. 91) | September 30, 1991 (30. 09. 91) |
| International Searching Authority | Signature of Authorized Officer |
| Japanese Patent Office | |

International Application No. PCT/JP91/00889

**FURTHER INFORMATION CONTINUED FROM THE SECOND SHEET**

| | | |
|---|---|---|
| Y | JP, B1, 48-38851 (Ryuzo Ueno), November 20, 1973 (20. 11. 73), (Family: none) | 1-4 |
| Y | JP, A, 62-106093 (Mitsui Toatsu Chemicals, Inc.), May 16, 1987 (16. 05. 87), (Family: none) | 1-4 |
| Y | JP, A, 60-8286 (Showa Denko K.K.), January 17, 1985 (17. 01. 85), (Family: none) | 1-4 |
| Y | JP, A, 58-10576 (Sanofi), | 1-4 |

**V.☐ OBSERVATIONS WHERE CERTAIN CLAIMS WERE FOUND UNSEARCHABLE [1]**

This international search report has not been established in respect of certain claims under Article 17(2) (a) for the following reasons:

1.☐ Claim numbers , because they relate to subject matter not required to be searched by this Authority, namely:

2.☐ Claim numbers , because they relate to parts of the international application that do not comply with the prescribed requirements to such an extent that no meaningful international search can be carried out, specifically:

3.☐ Claim numbers , because they are dependent claims and are not drafted in accordance with the second and third sentences of PCT Rule 6.4(a).

**VI.☐ OBSERVATIONS WHERE UNITY OF INVENTION IS LACKING [2]**

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

**FURTHER INFORMATION CONTINUED FROM THE SECOND SHEET**

| | | |
|---|---|---|
| | January 21, 1983 (21. 01. 83), & US, A, 4487931 & EP, B1, 68979 & FR, B1, 2508454 | |
| Y | JP, A, 53-50178 (SIGMA-TAU Industrie Farmaceutiche Riunite S.p.A.), May 8, 1978 (08. 05. 78), & US, A, 4167572 & FR, A1, 2367764 & GB, A, 1587273 | 1-4 |
| Y | JP, A, 60-188352 (Ludwig Heumann & Co., GmbH), September 25, 1985 (25. 09. 85), & US, A, 4659721 & DE, A1, 3404786 & EP, A1, 154721 | 1-4 |

**V.☐ OBSERVATIONS WHERE CERTAIN CLAIMS WERE FOUND UNSEARCHABLE [1]**

This international search report has not been established in respect of certain claims under Article 17(2) (a) for the following reasons:

1.☐ Claim numbers , because they relate to subject matter not required to be searched by this Authority, namely:

2.☐ Claim numbers , because they relate to parts of the international application that do not comply with the prescribed requirements to such an extent that no meaningful international search can be carried out, specifically:

3.☐ Claim numbers , because they are dependent claims and are not drafted in accordance with the second and third sentences of PCT Rule 6.4(a).

**VI.☐ OBSERVATIONS WHERE UNITY OF INVENTION IS LACKING [2]**

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

(Information concerning IPC to be added in the column I)

C07D405/12, C07D409/12, C07D417/12